# EUROPÄISCHE PATENTSCHRIFT

(11) **EP 2 493 617 B1**
(45) Veröffentlichungstag und Bekanntmachung des Hinweises auf die Patenterteilung: **20.02.2019**
(21) Anmeldenummer: 10760651.9
(22) Anmeldetag: 17.09.2010
(51) Int. Cl.: B01L 3/00, G01N 35/02

(54) **ANORDNUNG ZUR WAHLWEISEN DURCHFÜHRUNG EINES KLINISCH-CHEMISCHEN TESTS ODER EINES ELISA-TESTS**
ASSEMBLY FOR SELECTIVELY PERFORMING A CLINICAL CHEMICAL TEST OR AN ELISA TEST
SYSTÈME POUR LA MISE EN OEUVRE SÉLECTIVE D'UN TEST CLINICO-CHIMIQUE OU D'UN TEST ELISA

(30) Priorität: 30.10.2009 DE 102009051428
(43) Veröffentlichungstag der Anmeldung: 05.09.2012
(73) Patentinhaber: DRG Instruments GmbH, 35039 Marburg (DE)
(72) Erfinder: JANETZKO, Alfred, 35510 Butzbach (DE); SÄNGER, Wilhelm, 57234 Wilnsdorf (DE); GEACINTOV, Cyril, E., Mountainside, NJ 07092 (US)
(74) Vertreter: Patentanwälte Olbricht Buchhold Keulertz
(86) Internationale Anmeldenummer: PCT/EP2010/063732
(87) Internationale Veröffentlichungsnummer: WO 2011/051053

(56) Entgegenhaltungen:
- EP-A1- 1 650 570
- EP-A2- 1 255 115
- DE-A1-102004 054 551
- US-A- 4 608 231
- US-A- 5 482 861
- US-A- 6 143 250

## Beschreibung

Die Erfindung betrifft Anordnung zur wahlweisen Durchführung eines klinisch-chemischen Tests oder eines ELISA-Tests gemäß dem Oberbegriff von Anspruch 1 und die Verwendung der Anordnung gemäß dem nebengeordneten Anspruch 14 .

In der Fachwelt sind sowohl Analysevorrichtungen für die automatische Bestimmung von klinisch-chemischen als auch Anordnungen für die automatische Bestimmung von immundiagnostischen Parametern allgemein bekannt. Dabei werden jeweils unterschiedliche Bedingungen an Vorrichtungen zur Bestimmung klinisch-chemischer Parameter und an Vorrichtung zur Bestimmung immunologischer Parameter gestellt.

So werden klinisch-chemische Parameter in der Regel in der flüssigen Phase einer Probe bestimmt. Dazu wird der Probe zunächst wenigstens ein Reagenz zugefügt, welches eine Veränderung der Optischen Dichte bewirkt. Die Probe wird dann in eine Messküvette gegeben, die horizontal von einem optischen Messstrahl durchleuchtet wird. Die Reaktionstemperatur beträgt üblicherweise 37°C (= Körpertemperatur). Um den häufig aggressiven Reagenzien standhalten zu können, müssen die Reaktionsvorrichtungen aus entsprechend widerstandsfähigem Material gefertigt sein.

Immundiagnostische Parameter werden dagegen mit Hilfe einer an eine Festphase gekoppelten Substanz entsprechend dem ELISA-Prinzip bestimmt. Die Reaktionstemperatur beträgt dabei üblicherweise Raumtemperatur, d.h. 18°C bis 25°C. Kritisch ist bei diesen Tests insbesondere die Herstellung und Lagerung der antigen- bzw. antikörpergekoppelten Festphase. Dabei ist es insbesondere wichtig, die Antigene bzw. Antikörper optimal an die Festphase zu binden.

Weiterhin ist aus der US 5 482 861 A eine Anordnung zur Durchführung verschiedener Tests bekannt. Es werden insbesondere immunologische Tests wie *"fluorescent polarization immunoassays"* (FPIA) und *"microparticle enzyme immunoassays"* (MEIA) offenbart. Bei dieser Anordnung werden in ein Reaktionsbehälterkarussell verschiedene Reaktionsbehälter für die verschiedenen immunologischen Tests eingesetzt. Der Reaktionsbehälter zur Durchführung der Tests weist 7 Vertiefungen auf. Allerdings ist nur für die Durchführung eines FPIA-Tests, nicht aber für den MEIA-Test, eine Küvette vorgesehen.

EP 1 255 115 A2 sieht einen Analyseeinsatz zur einmaligen Verwendung bei der Durchführung eines ELISA in einer vollautomatischen Analysevorrichtung vor. Der Einsatz ist ein einteiliges Plastikformstück, in welchem mehrere Vertiefungen ausgebildet sind. Die erste Vertiefung nimmt die Probe auf, die weiteren Vertiefungen enthalten Reagenzien zur Durchführung des ELISA. In dem Gehäuse sind außerdem zwei Ausnehmungen ausgebildet, die durch einen Schlitz miteinander verbunden sind. In diese Ausnehmungen können nun zwei miteinander verbundene Wells eingesetzt werden, die zuvor aus einer mit entsprechendem Antigen oder Antikörper beschichteten Mikrotiterplatte ausgebrochen wurden.

Die zuvor genannten Lösungen haben jedoch mehrere Nachteile. So muss der Anwender beispielsweise die entsprechenden Wells selbst in den Analyseeinsatz einsetzen, was aufgrund der Architektur der Ausnehmung mehr oder weniger gut funktioniert. So besteht dabei beispielsweise auch die Gefahr, dass die in den übrigen Vertiefungen vorhandenen Reagenzien aus den Vertiefungen herausschwappen, wenn der Anwender eine entsprechend große Druckkraft ausüben muss, um das Well sicher in den Analyseansatz hineinzudrücken. Außerdem besteht die Gefahr, dass das Well zum Schluss in der Analysevorrichtung nicht korrekt positioniert ist, was im Ernstfall zu unerwünschten Lichtbrechungen und Ablenkungen des Messstrahles der Photometrischen Einrichtung führt und somit die Auswertung verfälschen kann. Zudem ist der Analyseeinsatz dadurch sehr fragil und es besteht bereits beim Abziehen der Sicherheitsfolie über den Reagenzienbeladenen Vertiefungen die Gefahr, dass der Einsatz derart verbogen wird, dass er nicht mehr in die Analysevorrichtung einsetzbar ist.

Ein weiterer Nachteil der liegt darin, dass der Analyseeinsatz nur in Vorrichtungen zur Durchführung von immundiagnostischen Analysen einsetzbar ist. Sofern es gewünscht ist, gleichzeitig klinisch-chemische Parameter zu bestimmen, muss der Anwender eine weitere, völlig anders aufgebaute Probe in eine weitere Vorrichtung einsetzen. Dies ist jedoch insbesondere in kleineren Arztpraxen nur kaum durchführbar, da dies zwingend sowohl die Anschaffung eines teuren Gerätes zur Analyse der immundiagnostischen Werte als auch die Anschaffung eines zweiten teuren Gerätes zur Analyse der klinisch-chemischen Parameter erfordert.

Aufgabe der Erfindung ist es daher, diese und weitere Nachteile des Standes der Technik zu überwinden und mit einer stets gleichen Anordnung von Reagenzienpatrone und Messküvetten eine Vielzahl von unterschiedlichen Parametern, vor allem sowohl immundiagnostische als auch klinisch-chemische, zu bestimmen und eine Reagenzienpatrone bereitzustellen, die kostengünstig und einfach herzustellen ist und die für den Anwender besonders einfach handhabbar ist.

Hauptmerkmale der Erfindung sind im kennzeichnenden Teil von Anspruch 1, sowie in Anspruch 13 angegeben. Ausgestaltungen sind Gegenstand der Ansprüche 2 bis 12.

Bei einer Anordnung zur wahlweisen Durchführung eines klinisch-chemischen Tests oder eines ELISA-Tests, umfassend eine Analysevorrichtung mit wenigstens einer Reagenzienpatrone und wenigstens einer Messküvette, wobei die Analysevorrichtung eine Probenaufnahme, eine Aufnahmevorrichtung für die Reagenzienpatronen und für die Messküvetten, und eine Pipettiervorrichtung aufweist, wobei die Aufnahmevorrichtung ein Karussell ist, wobei die Reagenzienpatronen und Messküvetten entlang von Radien des Karussells angeordnet sind, wobei die Reagenzienpatrone ein Gehäuse mit wenigstens einer Vertiefung umfasst, die eine Reaktions- oder Verdünnungskomponente enthält, und eine Ausnehmung aufweist, sieht die Erfindung vor, dass die Reagenzienpatrone drei Vertiefungen aufweist, wobei die erste Vertiefung der Reagenzienpatrone entweder Verdünnungslösung enthält oder ein zusätzliches Reagenz zur Durchführung eines klinisch-chemischen Tests, oder Enzymkonjugat zur Durchführung eines ELISA, oder Substrat zur Durchführung eines ELISA enthält oder leer ist, wobei die zweite Vertiefung entweder Verdünnungslösung oder ein Nachweisreagenz zur Durchführung eines klinisch-chemischen Tests oder Enzymkonjugat zur Durchführung eines ELISA, oder Substrat zur Durchführung eines ELISA enthält, oder leer ist und wobei die dritte Vertiefung entweder als Verdünnungsvertiefung dient oder, Verdünnungslösung oder ein Nachweisreagenz zur Durchführung eines klinisch-chemischen Tests enthält oder Enzymkonjugat zur Durchführung eines ELISA oder Substrat zur Durchführung eines ELISA, oder leer ist, wobei in der Aufnahmevorrichtung sowohl Vertiefungen zur Aufnahme von Messküvetten als auch Vertiefungen zur Aufnahme von Reagenzienpatronen ausgebildet sind, wobei jeweils eine Vertiefung zur Aufnahme einer Messküvette und eine Vertiefung zur Aufnahme einer Reagenzienpatrone auf einem gemeinsamen Radius der Aufnahemvorrichtung ausgebildet sind, wobei die Vertiefung zur Aufnahme der Messküvette am äußeren Ende des Radius angeordnet ist, wobei die Messküvetten an den äußeren Enden der Radien angeordnet sind, wobei die Reagenzienpatrone und die ihr zugeordnete Messküvettte linear in der Analysevorrichtung angeordnet sind, und wobei in die Ausnehmung des Gehäuses eine Festphase eingesetzt ist, an welche ein Antigen oder ein Antikörper koppelbar ist und wobei die Analysevorrichtung eine optische Einheit und eine Wascheinheit aufweist.

Die Erfindung sieht weiterhin eine Anordnung zur wahlweisen Durchführung eines klinisch-chemischen Tests oder eines ELISA-Tests umfassend eine Analysevorrichtung, wenigstens eine Reagenzienpatrone und wenigstens eine Messküvette, wobei die Reagenzienpatrone eine erfindungsgemäße Reagenzienpatrone entsprechend einem der Ansprüche 1 bis 11 ist und wobei jeder Reagenzienpatrone eine Messküvette zugeordnet ist, wobei die Reagenzienpatrone und die ihr zugeordnete Messküvettte 30 linear in der Analysevorrichtung angeordnet sind.

Bei einer solchen Anordnung ist es vorteilhaft, wenn die Analysevorrichtung eine Probenaufnahme, eine Aufnahmevorrichtung für die Reagenzienpatronen und für die Messküvetten und eine Pipettiervorrichtung aufweist und wenn die Aufnahmevorrichtung ein Karussell ist, wobei die Reagenzienpatronen und Messküvetten entlang der Radien des Karussells angeordnet sind und wobei die Messküvetten an den äußeren Enden der Radien angeordnet sind.

Man erkennt, dass der besondere Vorteil der erfindungsgemäßen Reagenzienpatrone darin liegt, dass sie sowohl zur Durchführung eines klinisch-chemischen Tests als auch eines immundiagnostischen Tests in ein und der selben Analysevorrichtung geeignet ist.

In einer ersten Ausführungsvariante ist es demgemäß vorteilhaft, dass die Reagenzienpatrone eine Reagenzienpatrone zur Durchführung eines klinisch-chemischen Tests ist, wobei die erste Vertiefung leer ist, die zweite Vertiefung ein erstes Nachweisreagenz und die dritte Vertiefung ein zweites Nachweisreagenz für die Durchführung eines klinisch-chemischen Tests enthält. Vorstellbar ist dabei auch, dass die Reagenzienpatrone eine Reagenzienpatrone zur Durchführung eines klinisch-chemischen Tests ist, wobei zwei der Vertiefungen leer sind und die dritte Vertiefung ein Nachweisreagenz für die Durchführung eines klinisch-chemischen Tests enthält. Dabei ist es besonders kostengünstig in der Herstellung, wenn die Festphase weder ein Antigen noch einen Antikörper gebunden hat und eine vierte, leere Vertiefung der Reagenzienpatrone darstellt.

In einer zweiten Ausführungsvariante ist vorgesehen, dass die Reagenzienpatrone eine Reagenzienpatrone zur Durchführung eines immundiagnostischen Tests ist, wobei die erste Vertiefung leer ist, die zweite Vertiefung Konjugat und die dritte Vertiefung Substrat zur Durchführung eines ELISA enthält, wobei an die Festphase ein Antigen oder Antikörper gekoppelt ist.

In einer weiteren Ausführungsvariante ist die Reagenzienpatrone eine Reagenzienpatrone zur Verdünnung einer Probe, wobei wenigstens eine der drei Vertiefungen eine Verdünnungslösung enthält und wenigstens eine der drei Vertiefungen als Verdünnungsvertiefung dient. Auch hier ist es in Hinblick auf Kosten und Herstellungsaufwand günstig, wenn an die Festphase weder ein Antigen noch ein Antikörper gebunden ist und dass die Festphase als Verdünnungsvertiefung dient oder leer ist.

Ein weiterer Vorteil der Erfindung liegt darin, dass die Festphase aus einem anderen Material als das Gehäuse gefertigt ist. So kann die Festphase aus Polystyrol gefertigt sein. Dieser Kunststoff begünstigt vor allem die Bindung des gewünschten Antigens oder Antikörpers an die Festphase. Gleichzeitig ist er allerdings nicht besonders resistent gegenüber vielen zur Durchführung von klinisch-chemischen Tests benötigten Reagenzien. Man erkennt daher den Vorteil, den es mit sich bringt, wenn das Gehäuse - wie oben genannt - aus einem anderen Material besteht. So ist es beispielsweise vorstellbar, dass das Gehäuse aus dem wesentlich resistenteren Werkstoff Polypropylen gefertigt ist. Auf diese Weise wird gleichzeitig sichergestellt, dass das Antigen oder der Antikörper sicher und beständig an die Festphase gekoppelt ist und dass die Reagenzien zur Durchführung klinisch-chemischer Tests dennoch gut in der Reagenzienpatrone lagerbar sind.

Vorteilhaft ist daher in wenigstens einer der Vertiefung ein Reagenz vorgelegt ist, während die Festphase entweder mit Antikörper geladen ist, leer ist oder als Verdünnungsvorrichtung dient.

Die Erfindung sieht zudem die Verwendung einer erfindungsgemäßen Reagenzienpatrone zur Analyse eines klinisch-chemischen Parameters in einer Probe, die Verwendung einer erfindungsgemäßen Reagenzienpatrone zur Analyse eines immundiagnostischen Parameters in einer Probe, die Verwendung einer erfindungsgemäßen Reagenzienpatrone als Verdünnungs-Vorrichtung und die Verwendung einer erfindungsgemäßen Reagenzienpatrone zur Bereitstellung von zusätzliche Reagenzien vor.

Eine solche Verwendung kann besonders vorteilhaft in einem Verfahren geschehen, das in einem Vollautomaten durchgeführt wird und die folgenden Schritte umfasst:
a) Einsetzen der Reagenzienpatronen für die durchzuführenden Analysen in eine Aufnahmevorrichtung des Vollautomaten
b) Einsetzen der Probe in eine Probenvorrichtung des Vollautomaten
c) Bestimmen der klinisch-chemischen Parameter mit Hilfe einer Messküvette, die der Reagenzienpatrone zugeordnet ist, die zum Durchführen der Analyse des jeweiligen klinisch-chemischen Parameters vorgesehen ist,
d) Bestimmen der immundiagnostischen Parameter mit Hilfe einer Messküvette, die der Reagenzienpatrone zugeordnet ist, die zum Durchführen der Analyse des jeweiligen immundiagnostischen Parameters vorgesehen ist,
e) Reinigen oder Entfernen der gebrauchten Messküvetten
f) Entfernen der verbrauchten Reagenzienpatronen und der Probengefäße.

Man erkennt, dass auf diese Weise in ein und demselben Verfahren sowohl klinisch-chemische Parameter als auch immunologische Komponenten einer Probe bestimmt werden können. Vor allem kann das Verfahren in ein und derselben Analysevorrichtung ausgeführt werden. Es ist daher nicht länger notwendig für die Analyse von klinisch-chemischen Parametern und immundiagnostischen Parametern verschiedene Apparate anzuschaffen, was insbesondere für kleinere Arztpraxen eine enorme Kostenersparnis darstellt. Dieser besondere Vorteil ergibt sich dadurch, dass sowohl die klinisch-chemischen Parameter mit Hilfe einer Messküvette als auch die immundiagnostischen Parameter mit Hilfe einer Messküvette bestimmt werden, wobei jeder Messküvette eine erfindungsgemäße Reagenzienpatrone zugeordnet ist. Dagegen werden bei allen herkömmlichen Verfahren lediglich die klinisch-chemischen Parameter mit einer Messküvette, die immundiagnostischen Parameter hingegen in einem Well einer Mikrotiterplatte gemessen.

Ein weiterer Vorteil liegt entsprechend darin, dass zur Auswertung sowohl der klinisch-chemischen Parameter als auch der immundiagnostischen Parameter nur eine einzige photometrische Einheit in der Analysevorrichtung, in der die erfindungsgemäße Reagenzienpatrone verwendet wird, vorhanden sein muss. Zugleich ist es möglich, dass in ein und demselben Analysegerät von einer Probe mehrere unterschiedliche klinisch-chemische und/oder immundiagnostische Parameter bestimmt werden. Ein Arzt kann daher beispielsweise zur Erstellung einer Diagnose wie folgt vorgehen: Zuerst nimmt er einem Patienten eine Probe der zu untersuchenden Körperflüssigkeit ab. Dann wählt er verschiedene erfindungsgemäße Reagenzienpatronen aus, je nachdem welche Parameter er bestimmen möchte. Für jeden der Parameter setzt er die entsprechenden Reagenzienpatronen in die Analysevorrichtung ein und startet damit das erfindungsgemäße Verfahren. Dieses liefert ihm, wie oben beschrieben die gewünschten unterschiedlichen klinisch-chemischen und immundiagnostischen Parameter, auf deren Grundlage er dann seine Diagnose stellen kann.

Man erkennt, dass die Verwendung der erfindungsgemäßen Reagenzienpatrone in einem solchen Verfahren, den Vorteil bietet, dass für jeden zu bestimmenden klinisch-chemischen oder immundiagnostischen Parameter eine eigene Reagenzienpatrone in eine Aufnahmevorrichtung für eine Reagenzienpatrone der Analysevorrichtung eingesetzt wird, wobei in der jeweiligen Reagenzienpatrone die für den jeweiligen Test notwendigen Nachweisreagenzien in Vertiefungen enthalten sind. Dabei ist es günstig, wenn die Reagenzienpatronen, die zur Analyse der klinisch-chemischen Parameter dienen, wenigstens ein Nachweisreagenz enthalten und wenn die Reagenzienpatronen, die zur Analyse der immundiagnostischen Parameter dienen, eine erste Vertiefung mit Konjugat, eine zweite Vertiefung mit Substrat und eine Festphase enthalten. Man erkennt, dass dies durch die erfindungsgemäße Reagenzienpatrone besonders vorteilhaft ermöglicht wird.

Das Bestimmen von immundiagnostischen Parametern kann dann beispielsweise die folgenden Schritte umfassen:
a) Aufnehmen von Emzymkonjugat aus einer ersten Vertiefung der zur Durchführung der immundiagnostischen Analyse vorgesehenen Reagenzienpatrone und Aufnehmen von Probe durch die Pipettiervorrichtung,
b) Abgeben von Enzymkonjugat und Probe aus der Pipettiervorrichtung auf die Festphase der Reagenzienpatrone,
c) Inkubieren der Festphase mit Konjugat und Probe,
d) Entfernen überschüssigen Konjugats und Probe durch Waschen der Festphase,
e) Aufnehmen von Substrat aus einer zweiten Vertiefung der zur Durchführung der immundiagnostischen Analyse vorgesehen Reagenzienpatrone durch die Pipettiervorrichtung,
f) Abgeben des Substrates aus der Pipettiervorrichtung auf die Festphase,
g) Inkubieren des Substrates auf der Festphase,
h) Aufnehmen des umgesetzten Substrates durch die Pipettiervorrichtung,
i) Abgegeben des umgesetzten Substrates aus der Pipettiervorrichtung in die Messküvette,
j) Messen der Konzentration des umgesetzten Substrates mit Hilfe der Messeinrichtung.

Alternativ ist es möglich, das Konjugat und die Probe in zwei separaten Schritten aufzunehmen und auf die Festphase zu pipettieren. Dabei kann beispielsweise erst das Konjugat durch die Pipettiervorrichtung aufgenommen und auf die Festphase gegeben werden, danach wird die Probe durch die Pipettiervorrichtung aufgenommen und auf die Festphase gegeben. Denkbar ist natürlich auch, dass zuerst die Probe aufgenommen und auf die Festphase gegeben wird und anschließend das Konjugat.

Dabei entsprechen die Schritte a) bis g) den allgemein gängigen Schritten zur Durchführung eines ELISA. Das bedeutet, bei der gemeinsamen Abgabe des Konjugates und der Probe auf die Festphase bindet der nachzuweisende immunologische Parameter, beispielsweise ein bestimmtes Peptid, ein Antikörper oder ein sonstiges Protein, einerseits an seinen an der Festphase gekoppelten Bindungspartner - also einen entsprechenden Antikörper, ein passendes Antigen o.ä. Andererseits enthält das Konjugat einen passenden Enzymkomplex, der aus einem Bindungspartner und einem an den Bindungspartner gekoppelten Enzym besteht. Der Bindungspartner bindet ebenfalls an den nachzuweisenden Parameter. Auf diese Weise wird der gesamte Komplex aus Parameter, Bindungspartner und Enzym an der Festphase immobilisiert. Überschüssiger Komplex und überschüssige Probe werden im nächsten Schritt abgenommen bzw. ausgewaschen. Die dann zugegebene Substratlösung enthält ein für das so an der Festphase immobilisierte Enzym spezifische Substrat. Dieses wird durch Reaktion mit dem Enzym umgesetzt, wodurch eine Farbveränderung und somit eine definierte Änderung der optischen Dichte bei einer bestimmten Wellenlänge hervorgerufen wird.

Der besondere Vorteil eines solchen Verfahrens bei der Verwendung der erfindungsgemäßen Reagenzienpatrone besteht nun darin, dass die Enzym-Substratreaktion dadurch beendet wird, dass die komplette Substratlösung, die nun sowohl umgesetztes als auch nicht-umgesetztes Substrat enthält, von der Festphase abgenommen und zur Auswertung in eine Messküvette umpipettiert wird. Auf diese Weise ist es möglich mit einer stets gleichen Anordnung von Reagenzienpatrone und Messküvetten eine Vielzahl von unterschiedlichen Parametern - vor allem sowohl immundiagnostische als auch klinisch-chemische - zu bestimmen. In einem stets gleichen Ablauf wird die Konzentration des entsprechenden Parameters mit Hilfe der Messküvette und einer einzigen photometrischen Einheit bestimmbar.

Ein weiterer Vorteil liegt darin, dass es nicht notwendig ist, als zusätzliche Komponente eine Stopp-Lösung bereitzustellen, die andernfalls üblicherweise verwendet wird, um die Enzym-Substratreaktion definiert zu beenden. Dieses Beenden erfolgt hier vielmehr einfach durch Abnehmen und Umpipettieren der teilweise umgesetzten Substratlösung in die Messküvette. Da die an der Festphase immobilisierten Enzyme dabei an der Festphase zurückbleiben und nicht in die Messküvette mitgenommen werden, erfolgt auch keine weitere Umsetzung des Substrates sobald die Lösung von der Festphase abgenommen wird.

Günstig ist es auch, wenn die Bestimmung der immundiagnostischen Parameter unter Verwendung der erfindungsgemäßen Reagenzienpatrone bei einer Temperatur zwischen 27°C und 39°C, bevorzugt bei einer Temperatur von 37°C, durchgeführt wird. Diese spezielle Angleichung der Temperaturbedingungen der ELISA-Tests ermöglicht es die klinisch-chemischen Parameter und die immundiagnostischen Parameter in beliebiger Reihenfolge unmittelbar nacheinander zu bestimmen, ohne jedes Mal die Temperatur des zur Analyse verwendeten Gerätes verändern zu müssen, was ansonsten mit langen Heiz- bzw. Kühlphasen und entsprechenden Wartezeiten verbunden wäre. Weitere Vorteile der Anhebung der Temperatur bestehen beispielsweise darin, dass die Temperatur deutlich oberhalb der üblichen Raumtemperatur liegt. Dadurch ist es unerheblich, wo das ausführende Gerät aufgestellt wird, die Temperatur bei der der Test durchgeführt wird, wird stets konstant gehalten. Aufgrund dessen sind die Reaktionskinetiken stets gleich. Dies führt beispielsweise zu einer höheren Reproduzierbarkeit der Testergebnisse. Auch kann auf das spezielle Anlegen einer Standardkurve für den einzelnen Test, wie sie ansonsten für ELISA üblich ist, verzichtet werden. Die in den erfindungsgemäßen Reagenzienpatronen bereitgestellten Komponenten zur Durchführung eines immundiagnostischen Tests sind daher mit besonderem Vorteil auf die Durchführung des Tests bei einer Temperatur von 37°C abgestimmt.

Bei einer solchen Anordnung ist es vorteilhaft, wenn die Analysevorrichtung eine optische Einheit und eine Wascheinheit aufweist. Des Weiteren ist es günstig, wenn die Probenaufnahme und die Wascheinheit von der Aufnahmevorrichtung getrennt ausgebildet sind,

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: Eine erfindungsgemäße Reagenzienpatrone
- Fig. 2: einen Querschnitt durch die Reagenzienpatrone von Fig. 1
- Fig. 3: eine Aufsicht auf die Reagenzienpatrone von Fig. 1
- Fig. 4a: Verwendung der Reagenzienpatrone aus Fig. 2 zur Durchführung eines ELISA
- Fig. 4b: Verwendung der Reagenzienpatrone aus Fig. 2 zur Durchführung eines klinisch-chemischen Tests
- Fig. 4c: Verwendung der Reagenzienpatrone aus Fig. 2 als Verdünnungspatrone
- Fig. 4d: Verwendung der Reagenzienpatrone aus Fig. 2 zur Bereitstellung zusätzlicher Reagenzien
- Fig. 5: die Verwendung einer erfindungsgemäßen Reagenzienpatrone in einem Vollautomaten
- Fig. 6: eine schematische Ansicht einer Anordnung zur wahlweisen Analyse klinisch-chemischer und immundiagnostischer Parameter in welcher erfindungsgemäße Reagenzienpatronen verwendet werden.

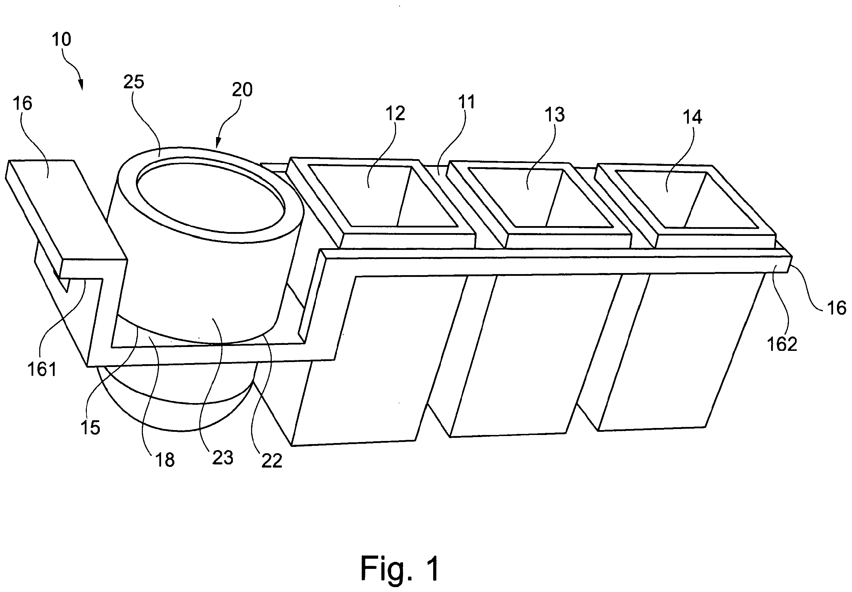

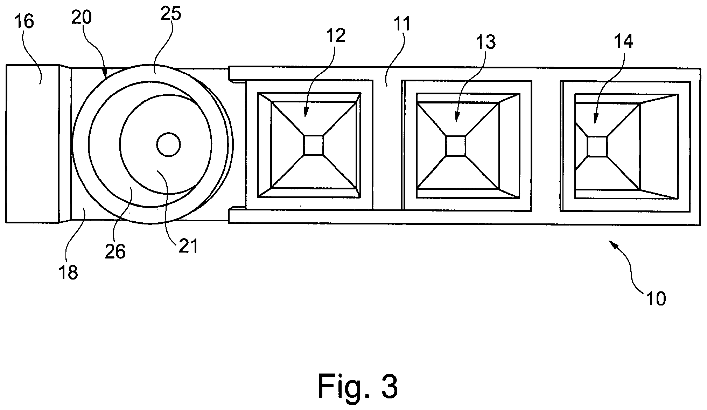

Die in den Figuren 1, 2 und 3 dargestellte erfindungsgemäße Reagenzienpatrone 10 besteht aus einem Gehäuse 11, in dem drei Vertiefungen 12, 13, 14 ausgebildet sind. In dem Gehäuse 11 ist außerdem eine Ausnehmung 15 ausgebildet, in welche eine Festphase 20 einsetzbar ist. Die Festphase 20 hat die Form eines einzelnen Wells einer Mikrotiterplatte und ist bevorzugt aus Polystyrol ausgebildet, während das Gehäuse 11 und die Vertiefungen 12, 13, 14 aus einem auf die Reagenzien der klinisch-chemischen Tests abgestimmten beständigen Material, beispielsweise Polypropylen, ausgebildet sind.

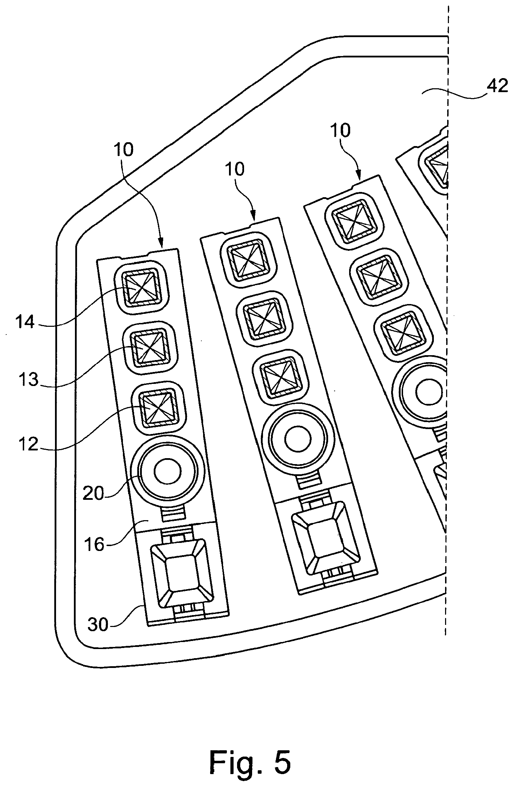

Das Gehäuse 11 hat eine Hauptebene 16 mit einem vorderen Ende 161 und einem hinteren Ende 162. Ist die Reagenzienpatrone 10 in die Analysevorrichtung eingesetzt, so liegt sie mit den Unterseiten des vorderen und hinteren Endes 161, 162 auf der Analysevorrichtung auf und ist auf diese Weise in ihrer Position gesichert (vgl. auch Fig. 5). Man erkennt, dass die oberen Öffnungen der Vertiefungen 12, 13, 14 in der oberen Ebene 16 ausgebildet sind. Die Ausnehmung 15, die zur Aufnahme der Festphase 20 dient, ist dagegen in einer tieferen Ebene 18 ausgebildet.

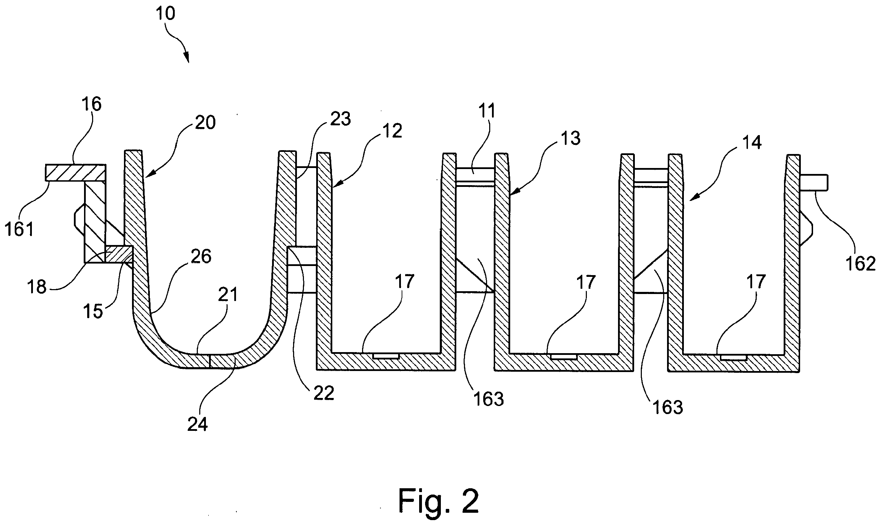

Die Festphase 20 ist in den Figuren 1 und 2 in die Ausnehmung 15 eingesetzt. Sie hat einen umlaufenden Rand 23 an dessen unteren Ende ein Vorsprung 22 ausgebildet ist. Mit diesem Vorsprung 22 liegt sie auf der Ebene 18 auf, so dass ein Hindurchrutschen durch die Ausnehmung 15 verhindert wird. Der oberen Rand 25 der Festphase 20 befindet sich auf gleicher Höhe mit den oberen Rändern der Vertiefungen 12, 13 und 14. Man erkennt, dass es ein weiterer besonderer Vorteil der erfindungsgemäßen Reagenzienpatrone 10 ist, dass diese bereits mit vorbereiteter und eingesetzter Festphase 20 an den Anwender geliefert werden kann. Die Festphase 20 ist darüber hinaus zuverlässig und sicher im Gehäuse 11 der Reagenzienpatrone 10 gelagert. Fehlern die durch Ungenauigkeiten des Anwenders beim Einsetzen des Festphase 20 in das Gehäuse 11 entstehen könnten, wird auf diese Weise wirksam vorgebeugt. Dies erleichtert nicht nur die Anwendung enorm, sondern führt auch zu einer Erhöhung der Zuverlässigkeit der mit Hilfe der Reagenzienpatrone 10 erzielten Messergebnisse.

Man erkennt insbesondere in Figur 2, dass zwischen den Vertiefungen 12, 13, 14 Stützstreben 163 ausgebildet sein können, die einen stabilen Gesamtaufbau der Reagenzienpatrone 10 sicherstellen. Vor allem gewährleisten die Stützstreben 163, dass die Reagenzienpatrone 10 im in die Analysevorrichtung eingesetzten Zustand stets plan liegt und sich nicht - beispielsweise durch Einwirkungen der Pipettiervorrichtung - verbiegen kann.

Ist die Reagenzienpatrone 10 zur Durchführung eines immundiagnostischen Tests gedacht, so kann an die Innenwand 21 des Bodens 24 und an die inneren Seitenwände 26 der Festphase 20 ein für den entsprechenden Test geeignetes Antigen oder ein entsprechender Antikörper gekoppelt werden. Ist die Reagenzienpatrone 10 zur Durchführung eines klinisch-chemischen Tests, zur Bereitstellung zusätzlicher Reagenzien oder als Verdünnungspatrone gedacht, so kann die Festphase 20 ohne daran gekoppelten Antikörper oder Antigen verwendet werden.

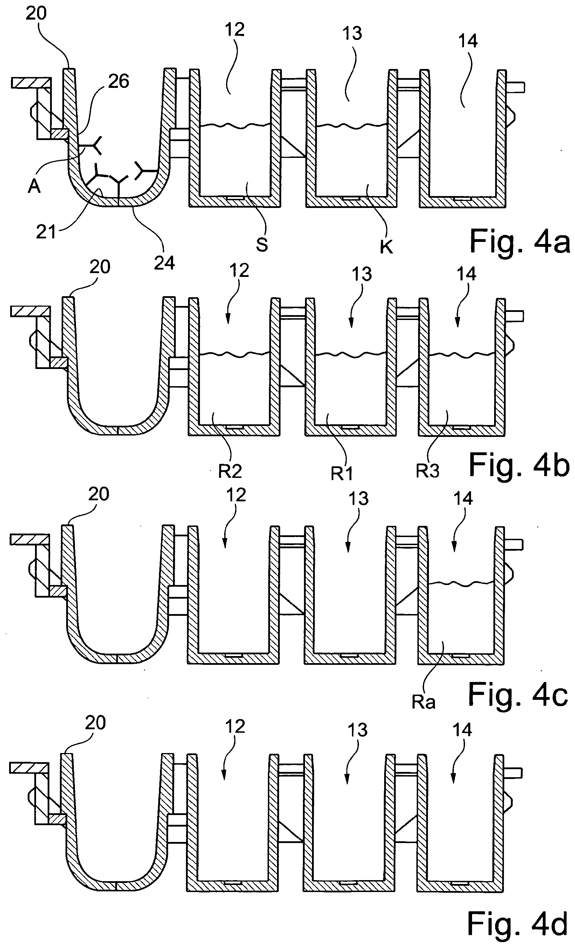

In den Figuren 4a bis 4d erkennt man verschiedene erfindungsgemäße Möglichkeiten, die Reagenzienpatrone 10 zu befüllen und diese im befüllten Zustand einem Anwender bereit zu stellen.

Die in Figur 4a dargestellte, erste Vertiefung 14 der Reagenzienpatrone 10 ist leer und kann für verschiedene Verwendungen flexibel genutzt werden. Die zweite Vertiefung 13 enthält ein Enzymkonjugat K und die dritte Vertiefung eine Substratlösung S. An den Boden 24 der Festphase 20 und an deren Seitenwände 26 ist ein Antigen oder Antikörper A gekoppelt.

Zur Durchführung eines ELISA mit Hilfe einer solchen Reagenzienpatrone werden mit einer (nicht vom Patent erfassten) Pippettiervorrichtung Enzymkonjugat K und die in einem separaten Gefäß außerhalb der Vorrichtung gelagerte (nicht dargestellte) Probe in die Festphase 20 gegeben. Dies kann entweder durch simultanes Aufnehmen des Enzymkonjugats K und der Probe P geschehen oder durch separate Pipettierschritte. Sofern die nachzuweisende immunologische Komponente in der Probe enthalten ist bindet diese einerseits an das auf der Festphase 20 immobilisierte Antigen oder den Antikörper A. Andererseits bindet die immunologische Komponente das Enzymkonjugat K. Auf diese Weise werden sowohl die nachzuweisende Komponente als auch das im Enzymkonjugat K enthaltene Enzym ebenfalls an der Festphase 20 immobilisiert. Überschüssige Probe und Enzymkonjugat K werden dann abgenommen und es wird die Substratlösung S in die Festphase 20 gegeben. Das in der Substratlösung S enthaltene Substrat wird von dem an der Festphase 20 immobilisierten Enzym des Enzymkonjugates K umgesetzt und sorgt auf diese Weise für eine photometrisch detektierbare Änderung der optischen Dichte der in der Festphase 20 enthaltenen Lösung.

Zur Durchführung eines klinisch-chemischen Tests wird die erfindungsgemäße Reagenzienpatrone 10 wie in Fig. 4b dargestellt nicht mit Enzymkonjugat und Substratlösung beladen, sondern mit einem ersten Reagenz R1 und sofern für den spezifischen Test notwendig einem oder zwei weiteren Reagenzien R2, R3 in den Vertiefungen 12, 13, 14 befüllt. Stattdessen ist es denkbar, dass auch die Festphase 20 ein Reagenz zur Durchführung des Tests aufnimmt. Man erkennt, dass ein besonderer Vorteil der erfindungsgemäßen Reagenzienpatrone 10 in deren vielseitigen Verwendbarkeit liegt. So ist es möglich je nach spezifischem Bedarf unterschiedliche Festphasen 20 in das Gehäuse 11 einzusetzen. Diese können sowohl aus einem der genannten Materialien als auch aus einem für die entsprechende Anwendung geeigneten dritten Material bestehen.

Werden für einen bestimmten klinisch-chemischen Test mehr als drei oder vier Reagenzien benötigt, so kann die erfindungsgemäße Reagenzienpatrone 10 auch dazu dienen, zusätzliche Reagenzien Ra aufzunehmen. Zur Durchführung des Tests wird die in Fig. 4c dargestellte Reagenzienpatrone 10 dann einfach neben der eigentlichen Reagenzienpatrone 10 wie sie in Fig. 4b dargestellt ist in die Analysevorrichtung eingesetzt.

Ist es für einen der gewünschten durchzuführenden Tests zudem notwendig, die zu analysierende Probe zu verdünnen, so kann eine weitere erfindungsgemäße Reagenzienpatrone 10 als Verdünnungspatrone verwendet werden. Bei dieser können wie in Fig. 4d dargestellt sowohl die Vertiefungen 12, 13, 14 als auch die Festphase 20 leer sein wobei die entsprechende Verdünnungslösung aus einem separat an der Analysevorrichtung vorgesehenen Gefäß zugegeben wird. Denkbar ist auch, dass in den als Verdünnungsvertiefungen dienenden Vertiefungen 12, 13, 14 eine Verdünnungslösung in definierten Volumina bereits vorgelegt ist.

In Fig.5 erkennt man, wie mehrere erfindungsgemäße Reagenzienpatronen 10 in einer vollautomatischen Analysevorrichtung 40 verwendet werden. Dabei sind sie derart in eine Karussellförmige Aufnahmevorrichtung 42 der Analysevorrichtung 40 eingesetzt, dass ihre Vertiefungen 12, 13, 14 und die Festphase 20 stets in einer Linie mit einer der jeweiligen Reagenzienpatrone 10 zugeordneten Messküvette 30, die zur photometrischen Auswertung der Analyseergebnisse dient, angeordnet sind.

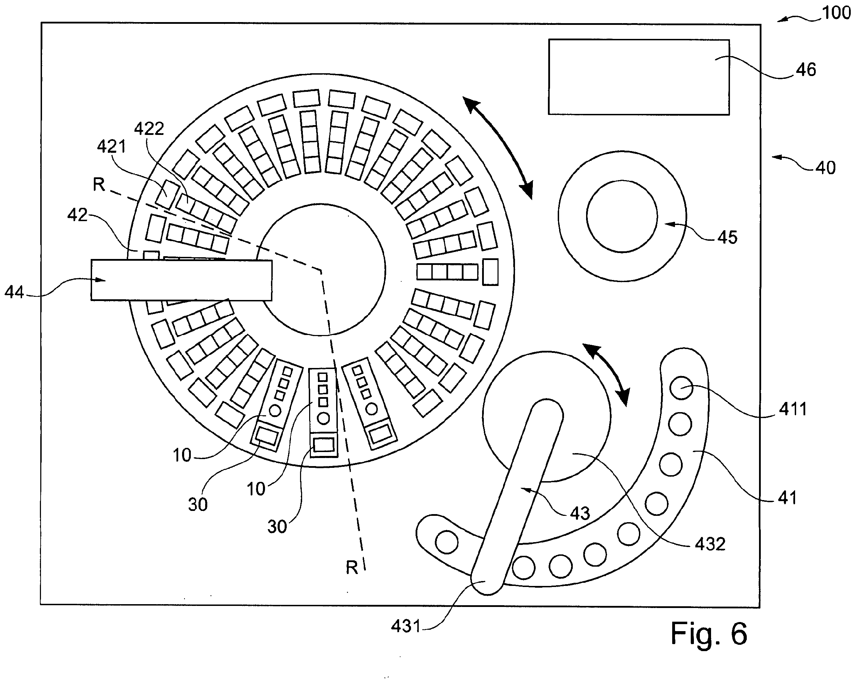

Eine Gesamtansicht der Analysevorrichtung 40 und mithin der Anordnung 100 erkennt man in Fig. 6. Die Analysevorrichtung 40 besteht aus einer Aufnahmevorrichtung 42, einer Probenaufnahme 41, einer Pipettiervorrichtung 43, einer optischen Einheit 44 und einer Wascheinheit 45.

In der Aufnahmevorrichtung 42 sind sowohl Vertiefungen 421 zur Aufnahme von Messküvetten 30 als auch Vertiefungen 422 zur Aufnahme von erfindungsgemäßen Reagenzienpatronen 10 ausgebildet. Man erkennt, dass jeweils eine Vertiefung 421 zur Aufnahme einer Messküvette 30 und eine Vertiefung zur Aufnahme einer Reagenzienpatrone 10 auf einem gemeinsamen Radius R der Aufnahemvorrichtung 42 ausgebildet sind, wobei die Vertiefung 421 zur Aufnahme der Messküvette 30 am äußeren Ende des Radius R angeordnet ist.

Auch in der Probenaufnahme 41 sind Vertiefungen 411 ausgebildet. In diese kann jeweils ein Röhrchen oder Cup welches die zu untersuchende Probe enthält eingesetzt werden. Man erkennt, dass die Probenaufnahme 41 von der Aufnahmevorrichtung 42 getrennt ausgebildet ist.

Die Pipettiervorrichtung 43 besteht aus einem schwenkbaren Roboterarm 431, der auf einer Schwenkvorrichtung 432 angebracht ist. An der Unterseite des vorderen Endes des Roboterarmes 431 ist eine Pipette angebracht, die in der Wascheinrichtung 45 bei Bedarf und zwischen den verschiedenen Pipettierschritten gereinigt werden kann.

Man erkennt, dass der Roboterarm 431 derart zwischen verschiedenen Positionen über der Probenaufnahme 41 und verschiedenen Positionen über der Aufnahmevorrichtung 42 schwenkbar ist, dass er gezielt entweder Reagenzien aus den eingesetzten Reagenzienpatronen 10 oder Proben aus der Probeaufnahme 41 aufnehmen und wahlweise in eine andere Vertiefung 12, 13, 14 einer Reagenzienpatrone 10, auf die Festphase 20 oder in die Messküvette 30 abgeben kann.

Im gezeigten Ausführungsbeispiel sind in einige der Vertiefungen 421, 422 der Aufnahmevorrichtung 42 Messküvetten 30 und Reagenzienpatronen 10 eingesetzt. Dabei zeigt sich ein besonderer Vorteil der Erfindung. Es ist nämlich nicht notwendig jeweils die komplette Aufnahmevorrichtung 42 zu bestücken. Ein Arzt, der die Anordnung zur schnellen Analyse der Probe eines Patienten nutzen möchte, geht vielmehr wie folgt vor:
Er überlegt, welche Parameter in der Probe er für seine Diagnose bestimmen möchte und nimmt dem Patienten die Probe ab. Er schaltet die Analysevorrichtung 40 ein, die sich daraufhin auf eine konstante Betriebstemperatur von 37°C erwärmt. Dann setzt er oder eine andere, den Test durchführende, Person die Probe in eine Vertiefung 411 der Probenaufnahme 41 und die den zu bestimmenden Parametern entsprechenden Reagenzienpatronen 10 nebst Messküvetten 30 in Vertiefungen 421, 422 der Aufnahmevorrichtung 42 ein. Als nächstes starte die durchführende Person über eine Computersteuerung 46 die Analyse. Die Computersteuerung 46 kann dabei entweder wie im gezeigten Ausführungsbeispiel ein unmittelbarer Bestandteil der Analysevorrichtung 40 sein oder ein externer an die Analysevorrichtung 40 angeschlossener Computer.

Der Roboterarm 43 nimmt dann entsprechend dem oben beschriebenen Verfahren Reagenzien und Proben auf und pipettiert diese so lange in entsprechender Weise hin und her bis in der Messküvette 30, die der Reagenzienpatrone 10 die dem jeweiligen zu bestimmenden Parameter zugeordnet ist, eine optisch auswertbare Lösung enthalten ist. Die Aufnahmevorrichtung 42 wird sodann derart gedreht, dass der Inhalt der Messküvette 30 in der optischen Einheit 44 ausgewertet werden kann. Bei der optischen Einheit 44 handelt es sich bevorzugt um eine photometrische Einheit, die bei einer für den jeweiligen Test vorgegebenen spezifischen Wellenlänge □ die optische Dichte OD_{□} der Lösung in der Messküvette 30 bestimmt und das Ergebnis an die Computereinheit zur Ausgabe weitergibt.

Besonders vorteilhaft ist es dabei, wenn bei der Analyse von immundiagnostischen Parametern mit Hilfe einer solchen Anordnung 100 und einer entsprechenden Analysevorrichtung 40 wie folgt vorgegangen wird:
a) Aufnehmen von Enzymkonjugat K aus einer ersten Vertiefung 13 der zur Durchführung der immundiagnostischen Analyse vorgesehenen Reagenzienpatrone 10 und Aufnehmen von Probe aus dem in eine Vertiefung 411 der Probenaufnahme 41 eingesetzten Probe durch die Pipettiervorrichtung 43,
b) Abgeben von Enzymkonjugat K und Probe aus der Pipettiervorrichtung 43 auf die Festphase 20 der Reagenzienpatrone 10,
c) Inkubieren der Festphase 20 mit Enzymkonjugat K und Probe,
d) Entfernen überschüssigen Enzymkonjugats K und Probe durch Waschen der Festphase 20,
e) Aufnehmen von Substrat S aus einer zweiten Vertiefung 12 der zur Durchführung der immundiagnostischen Analyse vorgesehen Reagenzienpatrone 10 durch die Pipettiervorrichtung 43,
f) Abgeben des Substrates S aus der Pipettiervorrichtung 43 auf die Festphase 20,
g) Inkubieren des Substrates S auf der Festphase 20,
h) Aufnehmen des umgesetzten Substrates S durch die Pipettiervorrichtung 43,
i) Abgegeben des umgesetzten Substrates S aus der Pipettiervorrichtung 43 in die Messküvette 30,
j) Messen der Konzentration des umgesetzten Substrates S mit Hilfe der optischen Einrichtung 44.

Dabei können selbstverständlich Konjugat K und Probe wie oben bereits dargestellt auch nach einander in beliebiger Reihenfolge aufgenommen und auf die Festphase 20 gegeben werden.

Zum Waschen der Festphase 20 wird entweder Waschlösung aus der Waschstation 45 in die Festphase 20 gespült oder es ist eine weitere (nicht dargestellte) Wascheinrichtung vorgesehen.

Die Erfindung ist nicht auf eine der vorbeschriebenen Ausführungsformen beschränkt, sondern in vielfältiger Weise abwandelbar.

So weist die Festphase 20 in den dargestellten Ausführungsbeispielen einen runden Boden und die Vertiefungen 12, 13, 14 jeweils einen flachen Boden 17 auf. Vorstellbar ist jedoch selbstverständlich auch eine andere Bodenarchitektur.

Sofern die Reagenzienpatrone 10 als Verdünnungspatrone dient, ist es auch vorstellbar, dass eine oder zwei der Vertiefungen 12, 13, 14 mit Verdünnungslösung beschickt sind, während die anderen Vertiefungen 12, 13, 14 leer sind, so dass in ihnen der tatsächliche Verdünnungsvorgang durch das Zugeben definierter Volumina von Verdünnungslösung und Probe durchgeführt werden kann.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

Bei einer Reagenzienpatrone 10 für eine Anordnung zur wahlweisen Durchführung eines klinisch-chemischen Tests oder eines ELISA-Tests, umfassend ein Gehäuse 11 mit wenigstens einer Vertiefung 12, 13, 14, die eine Reaktions- oder Verdünnungskomponente enthält, und eine Ausnehmung 15 aufweist, wobei in die Ausnehmung 15 des Gehäuses 11 eine Festphase 20 eingesetzt ist, an welche ein Antigen oder ein Antikörper koppelbar ist, ist es von besonderem Vorteil, wenn die Reagenzienpatrone 10 drei Vertiefungen 12, 13, 14 aufweist,
▪ wobei die erste Vertiefung 14 entweder als Verdünnungsvertiefung dient, Verdünnungslösung enthält oder ein zusätzliches Reagenz zur Durchführung eines klinisch-chemischen Tests enthält,
▪ wobei die zweite Vertiefung 13 entweder als Verdünnungsvertiefung dient, Enzymkonjugat K zur Durchführung eines ELISA oder ein Nachweisreagenz R1, R2, R3, Ra zur Durchführung eines klinisch-chemischen Tests enthält,
▪ wobei die dritte Vertiefung 12 entweder als Verdünnungsvertiefung dient, Substrat S zur Durchführung eines ELISA, ein Nachweisreagenz R1, R2, R3, Ra zur Durchführung eines klinisch-chemischen Tests enthält oder leer ist und
▪ wobei entweder alle Vertiefungen 12, 13, 14 der Reagenzienpatrone 10 als Verdünnungsvertiefung dienen und/oder Verdünnungslösung enthalten und/oder leer sind oder wobei die Vertiefungen 12, 13, 14 der Reagenzienpatrone 10 ein Nachweisreagenz R1, R2, R3, Ra zur Durchführung eines klinisch-chemischen Tests, Enzymkonjugat K zur Durchführung eines ELISA oder Substrat S zur Durchführung eines ELISA enthalten oder leer sind.

Wenn die Reagenzienpatrone 10 eine Reagenzienpatrone zur Durchführung eines klinisch-chemischen Tests ist, ist es zweckmäßig wenn die erste Vertiefung 14 leer ist, die zweite Vertiefung 13 ein erste Nachweisreagenz R1 und die dritte Vertiefung 12 ein zweites Nachweisreagenz R2 für die Durchführung eines klinisch-chemischen Tests enthält. Günstig ist es auch - sofern die Reagenzienpatrone 10 eine Reagenzienpatrone zur Durchführung eines klinisch-chemischen Tests ist - wenn zwei der Vertiefungen 12, 13 leer sind und die dritte Vertiefung 14 ein Nachweisreagenz Ra, R3 für die Durchführung eines klinisch-chemischen Tests enthält. Man erkennt dann ohne weiteres den Vorteil daran, dass die Festphase 20 weder ein Antigen noch einen Antikörper gebunden hat und eine vierte, leere Vertiefung der Reagenzienpatrone 10 darstellt.

Wenn die Reagenzienpatrone 10 eine Reagenzienpatrone zur Durchführung eines immundiagnostischen Tests ist, ist es zweckmäßig, dass die erste Vertiefung 14 leer ist, die zweite Vertiefung 13 Enzymkonjugat K und die dritte Vertiefung 12 Substrat S zur Durchführung eines ELISA enthält, wobei an die Festphase 20 ein Antigen oder Antikörper A gekoppelt ist.

Wenn die Reagenzienpatrone 10 eine Reagenzienpatrone zur Verdünnung einer Probe ist, ist es zweckmäßig, dass wenigstens eine der drei Vertiefungen 12, 13, 14 eine Verdünnungslösung enthält und wenigstens eine der drei Vertiefungen 12, 13, 14 als Verdünnungsvertiefung dient. Dann ist es vorteilhaft, wenn an die Festphase 20 weder ein Antigen noch ein Antikörper gebunden ist und wenn die Festphase 20 als Verdünnungsvertiefung dient oder leer ist.

Mit besonderem Vorteil erkennt man, dass die Festphase 20 aus einem anderen Material als das Gehäuse 11 gefertigt ist. Günstig ist es beispielsweise, wenn die Festphase 20 aus Polystyrol gefertigt ist.

Man erkennt, dass es bei einer erfindungsgemäßen Reagenzienpatrone 10 günstig ist, dass in wenigstens einer der Vertiefung 12, 13, 14 ein Reagenz R1, R2, R3, Ra, K, S vorgelegt ist, wobei die Festphase 20 entweder mit Antikörper A geladen ist, leer ist oder als Verdünnungsvorrichtung dient.

Man erkennt weiterhin den Vorteil der Verwendung einer erfindungsgemäßen Reagenzienpatrone 10, zur Analyse eines klinisch-chemischen Parameters in einer Probe, der Verwendung einer erfindungsgemäßen Reagenzienpatrone 10 zur Analyse eines immundiagnostischen Parameters in einer Probe, der Verwendung einer erfindungsgemäßen Reagenzienpatrone als Verdünnungs-Vorrichtung sowie der Verwendung einer erfindungsgemäßen Reagenzienpatrone 10 zur Bereitstellung von zusätzliche Reagenzien.

Die Erfindung sieht außerdem bei einer Anordnung 100 zur wahlweisen Durchführung eines klinisch-chemischen Tests oder eines ELISA-Tests umfassend eine Analysevorrichtung 40, wenigstens eine Reagenzienpatrone 10 und wenigstens eine Messküvette 30 vor, dass die Reagenzienpatrone eine erfindungsgemäße Reagenzienpatrone ist und dass jeder Reagenzienpatrone eine Messküvette zugeordnet ist, wobei die Reagenzienpatrone und die ihr zugeordnete Messküvettte 30 linear in der Analysevorrichtung angeordnet sind. Dabei ist es zweckmäßig, wenn die Analysevorrichtung 40 eine Probenaufnahme 41, eine Aufnahmevorrichtung 42 für die Reagenzienpatronen 10 und für die Messküvetten 30, und eine Pipettiervorrichtung 43 aufweist und wenn die Analysevorrichtung 40 eine optische Einheit 44 und eine Wascheinheit 45 aufweist. Vorteilhaft ist es, wenn dabei die Probenaufnahme 41 und die Wascheinheit 45 von der Aufnahmevorrichtung 42 getrennt ausgebildet sind und wenn die Aufnahmevorrichtung 42 ein Karussell ist, wobei die Reagenzienpatronen 10 und Messküvetten 30 entlang von Radien R des Karussells angeordnet sind und wobei die Messküvetten 30 an den äußeren Enden der Radien R angeordnet sind.

**Bezugszeichenliste**

| | | | |
|---|---|---|---|
| A | Antigen / Antikörper | 20 | Festphase |
| K | Enzymkonjugat | 21 | Innenwand |
| R | Radius | 22 | Vorsprung |
| R1 | Reagenz | 23 | Rand |
| R2 | Reagenz | 24 | Boden |
| R3 | Reagenz | 25 | Rand |
| Ra | Reagenz | 26 | Seitenwand |
| S | Substratlösung | | |
| | | 30 | Messküvette |
| 10 | Reagenzienpatrone | | |
| 11 | Gehäuse | 40 | Analysevorrichtung |
| 12 | Vertiefung | 41 | Probenaufnahme |
| 13 | Vertiefung | 411 | Vertiefung |
| 14 | Vertiefung | 42 | Aufnahmevorrichtung |
| 15 | Ausnehmung | 421 | Vertiefung |
| 16 | Hauptebene | 422 | Vertiefung |
| 161 | vorderes Ende | 43 | Pipettiervorrichtung |
| 162 | hinteres Ende | 431 | Roboterarm |
| 163 | Stützstrebe | 432 | Schwenkvorrichtung |
| 17 | Boden | 44 | optische Einheit |
| 18 | Ebene | 45 | Wascheinheit |
| | | 46 | Computersteuerung |

## Patentansprüche

1. Anordnung (100) zur wahlweisen Durchführung eines klinisch-chemischen Tests oder eines ELISA-Tests umfassend eine Analysevorrichtung (40), wenigstens eine Reagenzienpatrone (10) und wenigstens eine Messküvette (30),
a. wobei die Analysevorrichtung (40) eine Probenaufnahme (41), eine Aufnahmevorrichtung (42) für die Reagenzienpatronen (10) und für die Messküvetten (30) und eine Pipettiervorrichtung (43) aufweist,
b. wobei die Aufnahmevorrichtung (42) ein Karussell ist wobei die Reagenzienpatronen (10) und Messküvetten (30) entlang von Radien (R) des Karussells angeordnet sind,
c. wobei die Reagenzienpatrone (10) ein Gehäuse (11) mit wenigstens einer Vertiefung (12, 13, 14) umfasst, die eine Reaktions- oder Verdünnungskomponente enthält und eine Ausnehmung (15) aufweist,
**dadurch gekennzeichnet, dass**
d. die Reagenzienpatrone (10) drei Vertiefungen (12, 13, 14) aufweist,
o wobei die erste Vertiefung (14) der Reagenzienpatrone (10) entweder
I. Verdünnungslösung enthält, oder
II. ein zusätzliches Reagenz zur Durchführung eines klinisch-chemischen Tests, oder
III. Enzymkonjugat (K) zur Durchführung eines ELISA, oder
IV. Substrat (S) zur Durchführung eines ELISA enthält, oder
V. leer ist,
∘ wobei die zweite Vertiefung (13) entweder
I. Verdünnungslösung, oder
II. ein Nachweisreagenz (R1, R2, R3, Ra) zur Durchführung eines klinisch-chemischen Tests, oder
III. Enzymkonjugat (K) zur Durchführung eines ELISA, oder
IV. Substrat (S) zur Durchführung eines ELISA enthält, oder
V. leer ist,
∘ und wobei die dritte Vertiefung (12) entweder
I. als Verdünnungsvertiefung dient, oder
II. Verdünnungslösung, oder
III. ein Nachweisreagenz (R1, R2, R3, Ra) zur Durchführung eines klinisch-chemischen Tests enthält, oder
IV. Enzymkonjugat (K) zur Durchführung eines ELISA, oder
V. Substrat (S) zur Durchführung eines ELISA, oder
VI. leer ist,
e. wobei in der Aufnahmevorrichtung (42) sowohl Vertiefungen (421) zur Aufnahme der Messküvetten (30) als auch Vertiefungen (422) zur Aufnahme der Reagenzienpatronen (10) ausgebildet sind, wobei jeweils eine Vertiefung (421) zur Aufnahme der Messküvette (30) und eine Vertiefung zur Aufnahme einer Reagenzienpatrone (10) auf einem gemeinsamen Radius R der Aufnahemvorrichtung (42) ausgebildet sind, derart, dass die Reagenzienpatrone und die ihr zugeordnete Messküvettte (30) linear in der Analysevorrichtung angeordnet sind, wobei die Vertiefung (421) zur Aufnahme der Messküvette (30) am äußeren Ende des Radius R angeordnet ist,
f. wobei die Messküvetten (30) an den äußeren Enden der Radien (R) angeordnet sind,
g. wobei in die Ausnehmung (15) des Gehäuses (11) eine Festphase (20) eingesetzt ist, an welche ein Antigen oder ein Antikörper koppelbar ist
h. und wobei die Analysevorrichtung (40) eine optische Einheit (44) und eine Wascheinheit (45) aufweist.

2. Anordnung (100) nach Anspruch 1, **dadurch gekennzeichnet, dass** die Reagenzienpatrone (10) eine Reagenzienpatrone zur Durchführung eines klinisch-chemischen Tests ist, wobei die erste Vertiefung (14) leer ist, die zweite Vertiefung (13) ein erstes Nachweisreagenz (R1) und die dritte Vertiefung (12) ein zweites Nachweisreagenz (R2) für die Durchführung eines klinisch-chemischen Tests enthält.

3. Anordnung (100) nach Anspruch 1, **dadurch gekennzeichnet, dass** die Reagenzienpatrone (10) eine Reagenzienpatrone zur Durchführung eines klinisch-chemischen Tests ist, wobei zwei der Vertiefungen (12, 13) leer sind und die dritte Vertiefung (14) ein Nachweisreagenz (Ra, R3) für die Durchführung eines klinisch-chemischen Tests enthält.

4. Anordnung (100) nach einem der Ansprüche 1 bis 3, **dadurch gekennzeichnet, dass** die Festphase (20) weder ein Antigen noch einen Antikörper gebunden hat und eine vierte, leere Vertiefung der Reagenzienpatrone (10) darstellt.

5. Anordnung (100) nach Anspruch 1, **dadurch gekennzeichnet, dass** die Reagenzienpatrone (10) eine Reagenzienpatrone zur Durchführung eines immundiagnostischen Tests ist, wobei die erste Vertiefung (14) leer ist, die zweite Vertiefung (13) Enzymkonjugat (K) und die dritte Vertiefung (12) Substrat (S) zur Durchführung eines ELISA enthält, wobei an die Festphase (20) ein Antigen oder Antikörper (A) gekoppelt ist.

6. Anordnung (100) nach Anspruch 1 **dadurch gekennzeichnet, dass** die Reagenzienpatrone (10) eine Reagenzienpatrone zur Verdünnung einer Probe ist, wobei wenigstens eine der drei Vertiefungen (12, 13, 14) eine Verdünnungslösung enthält.

7. Anordnung (100) nach Anspruch 6, **dadurch gekennzeichnet, dass** an die Festphase (20) weder ein Antigen noch ein Antikörper gebunden ist und dass die Festphase (20) leer ist.

8. Anordnung (100) nach einem der Ansprüche 1 bis 7, **dadurch gekennzeichnet, dass** die Festphase (20) aus einem anderen Material als das Gehäuse (11) gefertigt ist.

9. Anordnung (100) nach einem der Ansprüche 1 bis 8, **dadurch gekennzeichnet, dass** die Festphase (20) aus Polystyrol gefertigt ist.

10. Anordnung (100) nach einem der Ansprüche 1 bis 9, **dadurch gekennzeichnet, dass** in wenigstens einer der Vertiefung (12, 13, 14) ein Reagenz (R1, R2, R3, Ra, K, S) vorgelegt ist.

11. Anordnung (100) nach einem der Ansprüche 1 bis 10, **dadurch gekennzeichnet, dass** die Festphase (20) entweder mit Antikörper (A) geladen ist oder leer ist.

12. Anordnung (100) nach Anspruch 1, **dadurch gekennzeichnet, dass** die Probenaufnahme (41) und die Wascheinheit (45) von der Aufnahmevorrichtung (42) getrennt ausgebildet sind.

13. Verwendung einer Anordnung (100) nach einem der Ansprüche 1 bis 12 **dadurch gekennzeichnet, dass** die Analyse von immundiagnostischen Parametern mit Hilfe der Anordnung (100) und einer Analysevorrichtung (40) erfolgt.

## Claims

1. Assembly (100) for selectively performing a clinical chemical test or an ELISA test comprising an analysis device (40), at least one reagent cartridge (10) and at least one measuring cuvette (30),
a. wherein the analysis device (40) has a sample mount (41), a mounting device (42) for the regent cartridges (10) and for the measuring cuvettes (30) and a pipette device (43),
b. wherein the mounting device (42) is a carousel, wherein the reagent cartridges (10) and measuring cuvettes (30) are arranged along radii (R) of the carousel,
c. wherein the reagent cartridge (10) comprises a housing (11) with at least one cavity (12, 13, 14) which contains a reaction or diluting component and has a recess (15),
**characterised in that**
d. the reagent cartridge (10) has three cavities (12, 13, 14),
∘ wherein the first cavity (14) of the reagent cartridge (10) contains either
I. diluting solution or
II. an additional reagent for performing a clinical chemical test, or
III. enzyme conjugate (K) for performing an ELISA, or
IV. substrate (S) for performing an ELISA or
V. is empty,
∘ wherein the second cavity (13) contains either
I. diluting solution or
II. a detection reagent (R1, R2, R3, Ra) for performing a clinical chemical test or
III. enzyme conjugate (K) for performing an ELISA or
IV. substrate (S) for performing an ELISA or
V. is empty,
∘ and wherein the third cavity (12) is either used
I. as a diluting cavity or contains
II. diluting solution or
III. a detection reagent (R1, R2, R3, Ra) for performing a clinical chemical test or
IV. enzyme conjugate (K) for performing an ELISA or
V. substrate (S) for performing an ELISA or
VI. is empty,
e. wherein in the mounting device (42) cavities (421) for mounting the measuring cuvettes (30) and also cavities (422) for mounting the reagent cartridges (10) are formed, wherein respectively a depression (421) is formed for mounting the measuring cuvette (30) and a depression is formed for mounting a reagent cartridge (10) on a common radius R of the mounting device (42), such that the reagent cartridge and the measuring cuvette (30) assigned thereto are arranged linearly in the analysis device, wherein the cavity (421) is arranged for receiving the measuring cuvette (30) at the outer end of the radius R,
f. wherein the measuring cuvettes (30) are arranged at the outer ends of the radii (R),
g. wherein in the recess (15) of the housing (11) a solid phase (20) is inserted to which an antigen or an antibody can be coupled,
h. and wherein the analysis device (40) has an optical unit (44) and a washing unit (45).

2. Assembly (100) according to claim 1, **characterised in that** the reagent cartridge (10) is a reagent cartridge for performing a clinical chemical test, wherein the first cavity (14) is empty, the second cavity (13) contains a first detection reagent (R1) and the third cavity (12) contains a second detection reagent (R2) for performing a clinical chemical test.

3. Assembly (100) according to claim 1, **characterised in that** the reagent cartridge (10) is a reagent cartridge for performing a clinical chemical test, wherein two of the cavities (12, 13) are empty and the third cavity (14) contains a detection reagent (Ra, R3) for performing a clinical chemical test.

4. Assembly (100) according to any of claims 1 to 3, **characterised in that** the solid phase (20) has neither an antigen nor an antibody bound to it and represents a fourth cavity of the reagent cartridge (10) which is empty.

5. Assembly (100) according to claim 1, **characterised in that** the reagent cartridge (10) is a reagent cartridge for performing an immunodiagnostic test, wherein the first cavity (14) is empty, the second cavity (13) contains enzyme conjugate (K) and the third cavity (12) contains substrate (S) for performing an ELISA, wherein an antigen or antibody (A) is coupled to the solid phase (20).

6. Assembly (100) according to claim 1, **characterised in that** the reagent cartridge (10) is a reagent cartridge for diluting a sample, wherein at least one of the three cavities (12, 13, 14) contains a diluent solution.

7. Assembly (100) according to claim 6, **characterised in that** neither an antigen nor an antibody is bound to the solid phase (20) and the solid phase (20) is empty.

8. Assembly (100) according to any of claims 1 to 7, **characterised in that** the solid phase (20) is made from a different material than the housing (11).

9. Assembly (100) according to any of claims 1 to 8, **characterised in that** the solid phase (20) is made of polystyrene.

10. Assembly (100) according to any of claims 1 to 9, **characterised in that** a reagent (R1, R2, R3, Ra, K, S) is pre-filled in at least one of the cavities (12, 13, 14).

11. Assembly (100) according to any of claims 1 to 10, **characterised in that** the solid phase (20) is either charged with antibody (A) or is empty.

12. Assembly (100) according to claim 1, **characterised in that** the sample mount (41) and the washing unit (45) are designed to be separate from the mounting device (42).

13. Use of an assembly (100) according to any of claims 1 to 12, **characterised in that** the analysis of immunodiagnostic parameters is performed by means of the assembly (100) and an analysis device (40).

## Revendications

1. Agencement (100) pour effectuer sélectivement un test de chimie clinique ou un test ELISA comprenant un dispositif d'analyse (40), au moins une cartouche de réactif (10) et au moins une cuvette de mesure (30),
a. ²dans lequel le dispositif d'analyse (40) présente un récepteur d'échantillon (41), un dispositif de réception (42) pour les cartouches de réactif (10) et pour les cuvettes de mesure (30) et un dispositif de prélèvement par pipette (43),
b. dans lequel le dispositif de réception (42) est un carrousel, dans lequel les cartouches de réactif (10) et les cuvettes de mesure (30) sont agencées le long de rayons (R) du carrousel,
c. dans lequel la cartouche de réactif (10) comprend un boîtier (11) comportant au moins une cavité (12, 13, 14), qui contient un composant de réaction ou de dilution et présentant un évidement (15),
**caractérisé en ce que**
d. la cartouche de réactif (10) présente trois cavités (12, 13, 14),
∘ dans lequel la première cavité (14) de la cartouche de réactif (10)
I. contient une solution de dilution, ou
II. contient un réactif supplémentaire pour la réalisation d'un test de chimie clinique, ou
III. un conjugué enzymatique (K) pour la réalisation d'un ELISA, ou
IV. un substrat (S) pour effectuer un ELISA, ou
V. est vide
∘ dans lequel la deuxième cavité (13)
I. contient une solution de dilution, ou
II. un réactif de détection (R1, R2, R3, Ra) pour la réalisation d'un test de chimie clinique, ou
III. un conjugué enzymatique (K) pour la réalisation d'un ELISA, ou
IV. un substrat (S) pour effectuer un ELISA, ou
V. est vide
∘ et dans lequel la troisième cavité (12)
I. contient une solution de dilution, ou
II. un réactif de détection (R1, R2, R3, Ra) pour la réalisation d'un test de chimie clinique, ou
III. un conjugué enzymatique (K) pour la réalisation d'un ELISA, ou
IV. un substrat (S) pour effectuer un ELISA, ou
V. est vide
e. dans lequel, dans le dispositif de réception (42), sont également formées des cavités (421) destinées à recevoir les cuvettes de mesure (30) ainsi que des cavités (422) destinées à recevoir les cartouches de réactif (10), dans lequel respectivement une cavité (421) destinée à recevoir la cuvette de mesure (30) et une cavité destinée à recevoir une cartouche de réactif (10) sont formées sur un rayon commun R du dispositif de réception (42), de sorte que la cartouche de réactif et sa cuvette de mesure associée (30) sont disposées de manière linéaire dans le dispositif d'analyse, dans lequel la cavité (421) destinée à recevoir la cuvette de mesure (30) est disposée au niveau de l'extrémité extérieure du rayon R,
f. dans lequel les cuvettes de mesure (30) sont disposées au niveau des extrémités extérieures des rayons (R),
g. dans lequel dans l'évidement (15) du boîtier (11) est disposée une phase solide (20) à laquelle peut être couplé un antigène ou un anticorps,
h. et dans lequel le dispositif d'analyse (40) comprend une unité optique (44) et une unité de lavage (45).

2. Agencement (100) selon la revendication 1, **caractérisé en ce que** la cartouche de réactif (10) est une cartouche de réactif pour la réalisation d'un test de chimie clinique, dans lequel la première cavité (14) est vide, la deuxième cavité (13) contient un premier réactif de détection (R1) et la troisième cavité (12) contient un second réactif de détection (R2) pour effectuer un test de chimie clinique.

3. Agencement (100) selon la revendication 1, **caractérisé en ce que** la cartouche de réactif (10) est une cartouche de réactif destinée à effectuer un test de chimie clinique, dans lequel deux des cavités (12, 13) sont vides et la troisième cavité (14) contient un réactif de détection (Ra, R3) pour la réalisation un test de chimie clinique.

4. Agencement (100) selon l'une des revendications 1 à 3, **caractérisé en ce que** la phase solide (20) n'a pas d'antigène ni d'anticorps lié, et constitue une quatrième cavité vide de la cartouche de réactif (10).

5. Agencement (100) selon la revendication 1, **caractérisé en ce que** la cartouche de réactif (10) est une cartouche de réactif pour effectuer un test d'immuno-diagnostique, dans lequel la première cavité (14) est vide, la deuxième cavité (13) contient le conjugué enzymatique (K) et la troisième cavité (12) contient un substrat (S) pour effectuer un ELISA, dans lequel un antigène ou anticorps (A) est couplé à la phase solide (20).

6. Agencement (100) selon la revendication 1, **caractérisé en ce que** la cartouche de réactif (10) est une cartouche de réactif pour la dilution d'un échantillon, dans lequel au moins l'une des trois cavité (12, 13, 14) contient une solution de dilution.

7. Agencement (100) selon la revendication 6, **caractérisé en ce que** ni un antigène ni un anticorps n'est lié à la phase solide (20), et **en ce que** la phase solide (20) est vide.

8. Agencement (100) selon l'une des revendications 1 à 7, **caractérisé en ce que** la phase solide (20) est réalisée dans un matériau différent de celui du boîtier (11).

9. Agencement (100) selon l'une des revendications 1 à 8, **caractérisé en ce que** la phase solide (20) est en polystyrène.

10. Agencement (100) selon l'une des revendications 1 à 9, **caractérisé en ce qu'**un réactif (R1, R2, R3, Ra, K, S) est fourni dans au moins une des cavités (12, 13, 14).

11. Agencement (100) selon l'une des revendications 1 à 10, **caractérisé en ce que** la phase solide (20) est soit chargée d'anticorps (A), soit vide.

12. Agencement (100) selon la revendication 1, **caractérisé en ce que** le récepteur d'échantillon (41) et l'unité de lavage (45) sont formés séparément du dispositif de réception (42).

13. Utilisation d'un agencement (100) selon l'une quelconque des revendications 1 à 12, **caractérisée en ce que** l'analyse de paramètres d'immuno-diagnostique survient à l'aide de l'agencement (100) et d'un dispositif d'analyse (40).
